# EUROPÄISCHE PATENTSCHRIFT

(11) **EP 1 139 923 B1**
(45) Veröffentlichungstag und Bekanntmachung des Hinweises auf die Patenterteilung: **17.07.2002**
(21) Anmeldenummer: 99947303.6
(22) Anmeldetag: 10.09.1999
(51) Int. Cl.: A61F 2/28, A61F 2/80

(54) **OBERSCHENKELSTUMPF-ENDOPROTHESE FÜR EINE EXOPROTHETISCHE VERSORGUNG**
UPPER LEG STUMP ENDOPROSTHESIS FOR AN EXOPROSTHETIC PROVISION
ENDOPROTHESE D'UN MOIGNON DE LA CUISSE POUR SOINS EXOPROTHETIQUES

(30) Priorität: 16.12.1998 DE 19857907
(43) Veröffentlichungstag der Anmeldung: 10.10.2001
(73) Patentinhaber: ESKA Implants GmbH & Co., 23556 Lübeck (DE)
(72) Erfinder: GRUNDEI, Hans, D-23558 Lübeck (DE)
(74) Vertreter: Weiss, Christian, Dipl.-Ing.
(86) Internationale Anmeldenummer: EP9906676
(87) Internationale Veröffentlichungsnummer: WO0035380

(56) Entgegenhaltungen:
- DE-A- 4 338 746
- DE-U- 9 400 720
- US-A- 4 158 895

## Beschreibung

Die Erfindung betrifft eine Oberschenkelstumpf-Endoprothese für eine exoprothetische Versorgung eines im Oberschenkelbereich beinamputierten Patienten.

Eine Prothese gemäß dem Oberbegriff von Anspruch 1 ist aus der DE-A-4 338 746 bekannt.

Üblicherweise wird über den Oberschenkelstumpf des zu versorgenden Patienten ein Trichter bzw. Schaft, beispielsweise aus zähelastischem Kunststoff gezogen, an dem die weiteren Teile der Prothese wie Kniegelenk-, Unterschenkel- und Fußteil angebracht sind.

Ein großes Problem dabei ist, wie die Belastungskräfte über das Gewebe in den Femurstumpf eingeleitet werden können. Zwischen dem Femurstumpf und dem äußeren des Oberschenkelstumpfes befindet sich nämlich Muskelgewebe, welches bei einer Bewegung ständig hohen Belastungen ausgesetzt ist, ohne daß das Wahrnehmungsgefühl des Patienten hierdurch gefördert wird in Richtung auf den natürlichen Gehkontakt.

Aus den Druckschriften DE-A-43 38 746 und DE-C-31 25 268 sind nun Femur-Knochenstumpfimplantate bekannt, welche das angesprochene Problem dadurch versuchen zu lösen, daß in den Femurstumpf ein Stielteil gesetzt wird, welches auf Dauer im Knochenkanal fixiert wird. Zum anderen Ende hin weisen die Implantate pilzkopfartig vergrößerte Kopfenden auf, welche die Belastungskräfte auf größere Flächen verteilen sollen. Diese Art der Versorgung verspricht einen besseren Tragekomfort gegenüber dem bislang üblichen Versorgungsmöglichkeiten, wenn auch das Wahrnehmungsgefühl bei dem damit versorgten Patienten mit dem natürlichen Wahrnehmungsgefühl vor der Amputation praktisch nichts gemein hat.

Vor diesem Hintergrund ist es die Aufgabe der vorliegenden Erfindung, eine eingangs erwähnte Oberschenkelstumpf-Endoprothese so weiterzubilden, daß das Wahrnehmungsgefühl des Patienten erheblich gesteigert wird und so das Gehverhalten als natürlicher empfunden wird.

Gelöst wird die Aufgabe durch eine Oberschenkelstumpf-Endoprothese mit den Merkmalen des Anspruchs 1. Weitere vorteilhafte Ausführungsformen ergeben sich aus den Unteransprüchen.

Demgemäß ist vorgesehen, daß die Endoprothese ein proximales Stielteil aufweist, das in einen Femurstumpf setzbar ist, wobei das Stielteil zumindest teilweise mit einer offenmaschigen dreidimensionalen Raumnetzstruktur bedeckt ist und an seinem distalen Ende einen konischen Adapter aufweist, mittels dessen ein metallischer Kondylenersatz, der der Form natürlicher Kondylen eines Kniegelenks nachgebildet ist, an das Stielteil gekoppelt ist.

Die offenmaschige, dreidimensionale Raumnetzstruktur, die auch als interkonnektierend bezeichnet wird, ermöglicht ein Ein-, Durch-, Hinter- und Umwachsen von natürlichem Knochenmaterial während der Einheilphase, so daß der Stielteil nach relativ kurzer Zeit - jedenfalls im Hinblick auf den Substratfluß - im Röhrenknochen integriert wird und eine extrem stabile Sekundärfixation gewährleistet ist. Das Einwachsen von Knochenmaterial in eine offenmaschige, dreidimensionale Raumnetzstruktur ist an sich bekannt aus der reinen Endoprothetik. Hier sei beispielsweise auf die DE-PS 41 06 971 hingewiesen.

Der distalseitig vorgesehene konische Adapter erlaubt die Ankopplung eines massiven Teils, nämlich des Kondylenersatzes. Der konische Adapter besteht dabei aus einer konischen Klemmhülse und einem konischen Klemmzapfen, wobei erstere beispielsweise im Stielteil eingearbeitet ist und der Zapfen am Kondylenersatz angeformt ist.

Durch den Kondylenersatz werden die Druckkräfte großflächiger verteilt und vor allem durch die Nachbildung der natürlichen Kondylen wird ein natürlicheres Wahrnehmungsvermögen des Patienten, welches unter der Bezeichnung "Osteoperzeption" bekannt ist, erzielt.

Diese wird noch dadurch gesteigert, daß die Innenauskleidung des Schaftes, der über den Oberschenkelstumpf gestülpt wird, die Form natürlicher Gleitkufen eines Femurteils eines natürlichen Kniegelenkes nachempfindet. Die den natürlichen Kondylen nachempfundene Formgebung des Kondylenersatzes dient als Klemmvorrichtung für die nachempfundenen Gleitkufen und erhöht so die Kontakthaftung des Stumpfes des Patienten beim Laufen erheblich.

Gemäß einer vorteilhaften Weiterbildung besteht der Kondylenersatz aus einem metallischen Grundkörper, der mit einer stoßdämpfenden Schicht aus Kunststoff beschichtet ist. Durch die stoßdämpfende Schicht gelangen Stoßbelastungen nicht ungedämpft in den verbliebenen Femurstumpf. Auch hierdurch kann die Osteoperzeption verbessert werden, da auch bei einem gesunden Patienten Belastungen auf das Sprung- und auf das Kniegelenk nicht ungedämpft auf den Femurknochen eingeleitet werden.

Hierzu weist die stoßdämpfende Schicht vorzugsweise eine Dicke von 3 bis 10mm auf.

Gemäß einer besonders bevorzugten Ausführungsform ist vorgesehen, daß die stoßdämpfende Schicht aus Silikon besteht. Dieses weist von sich aus eine gewisse Zähelastizität und Pufferwirkung auf, welche sich hervorragend für den Einsatz im Rahmen der vorliegenden Erfindung eignet.

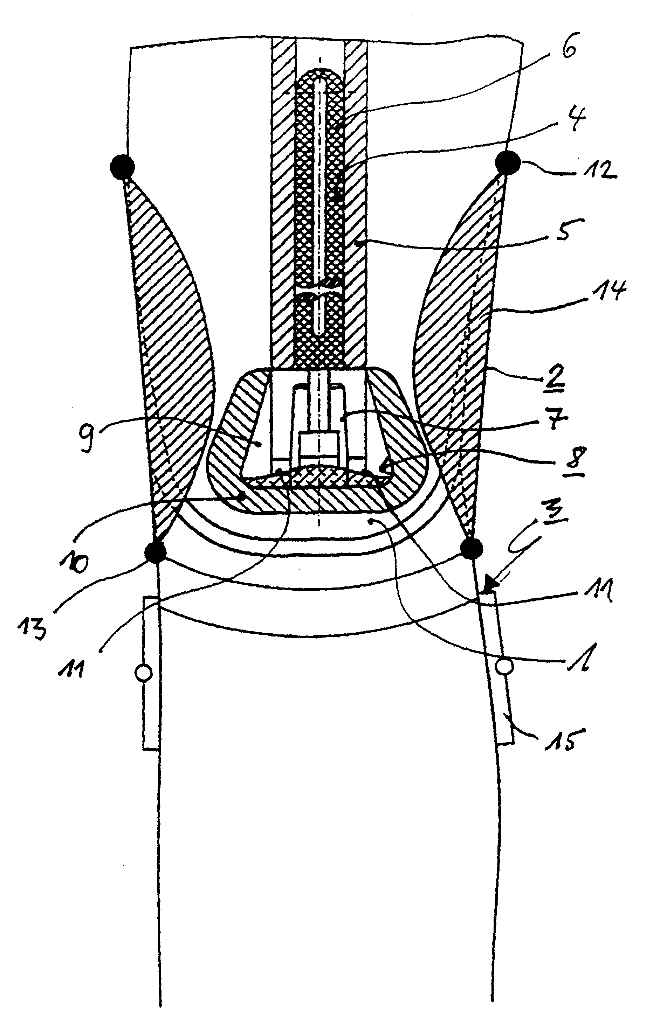

Die Erfindung wird nachfolgend anhand der einzigen Zeichnungsfigur näher erläutert.

Diese zeigt schematisch einen Oberschenkelstumpf 1 mit einer in dem Femurstumpf 5 implantierten Oberschenkelstumpf-Endoprothese.

Ein intramedullärer Stiel 4 bildet daß proximale Stielteil, welches in den Markraum des Femurstumpfes 5 gesetzt ist. Die Oberfläche des Stielteils 4 ist mit einer offenmaschigen, dreidimensionalen Raumnetzstruktur 6 belegt, durch welche hindurch Knochentrapekel wachsen können, so daß das Stielteil 4 nach einer gewissen Einheilphase, was den Substratfluß betrifft, quasi Bestandteil des natürlichen Knochens geworden ist.

Dem Stielteil 4 schließt sich distalseitig ein konischer Adapter 7 an. Mittels dieses konischen Adapters 7 ist distalseitig der Kondylenersatz 11 mit dem Stielteil 4 gekoppelt.

Das Kondylenersatzteil 8 ist so ausgebildet, daß es die Form 11 natürlichen Kondylen eines Kniegelenkes nachempfindet.

Der Kondylenersatz 8 ist in dem gezeigten Ausführungsbeispiels mit einer stoßdämpfenden Schicht 10 aus Silikon beschichtet.

Die gesamte Oberschenkelstumpf-Endoprothese ist also innerhalb des Oberschenkels plaziert. Es findet kein Durchtritt an irgendeiner Stelle des Oberschenkelstumpfes statt.

Der Oberschenkelstumpf 1 wird in einen Oberschenkel-Schaft 2 gezogen, an welchem nachgebildete Knie-, Unterschenkel- und Fußteile 3 angebracht sind.

Das Kondylenteil 8 mit seiner natürlichen Kondylen nachempfindenden Form 11 findet seinen Widerpart in einer entsprechenden Ausbildung des Inneren des Schaftes 2, das den natürlichen Gleitkufen eines Kniegelenks nachempfunden ist.

Der Oberschenkelschaft 2 ist vorliegend gebildet aus lateral und medial an dem Oberschenkelstumpf anliegenden Druckpoister 14, die durch eine Verschlußklemme 12 sowie durch ein schwenkbares Scharnier 13 fest am Oberschenkelstumpf arretiert sind.

Die Kniegelenkfunktion als solche wird ausgeübt durch ein künstliches Kniegelenk 15, wie es beispielsweise bekannt ist aus der EP-C-0 358 056.

## Patentansprüche

1. Oberschenkelstumpf-Endoprothese für eine exoprothetische Versorgung eines im Oberschenkelbereich beinamputierten Patienten, wobei der Oberschenkelstumpf (1) in einen Schaft (2) ziehbar ist, dem sich die Nachbildung eines Kniegelenk-, Unterschenkel- und Fußteils (3) der Prothese anschließt, aufweisend ein proximales Stielteil (4), das in einen Femurstumpf (5) setzbar ist, wobei das Stielteil (4) zumindest teilweise mit einer offenmaschigen, dreidimensionalen Raumnetzstruktur (6) bedeckt ist **dadurch gekennzeichnet, daß** das Stielteil an seinem distalen Ende einen konischen Adapter (7) aufweist, mittels dessen ein Kondylenersatz (8), der der Form (11) natürlicher Kondylen eines Kniegelenks nachgebildet ist, an das Stielteil (4) gekoppelt ist.

2. Oberschenkelstumpf-Endoprothese nach Anspruch 1, bei der der Kondylenersatz (8) aus einem metallischen Grundkörper (9) besteht, der mit einer stoßdämpfenden Schicht (10) aus Kunststoff beschichtet ist.

3. Oberschenkelstumpf-Endoprothese nach Anspruch 2, bei der die stoßdämpfende Schicht (10) eine Dicke von 3 bis 10 mm aufweist.

4. Oberschenkelstumpf-Endoprothese nach Anspruch 2 oder 3, bei der die stoßdämpfende Schicht (10) aus Silikon besteht.

## Claims

1. Thigh stump endoprosthesis for exoprosthetic care of a patient with leg amputation in the thigh region, wherein the thigh stump (1) can be drawn into a shaft (2), to which the reproduction of a knee joint, lower leg and foot part (3) of the prosthesis is connected, having a proximal stem part (4), which can be placed in a femoral stump (5), wherein the stem part (4) is covered at least partly by an open-meshed, three-dimensional spatial network structure (6), **characterised in that** the stem part has at its distal end a conical adapter (7), by means of which a condyle replacement (8), which reproduces the shape (11) of natural condyles of a knee joint, is coupled to the stem part (4).

2. Thigh stump endoprosthesis according to claim 1, in which the condyle replacement (8) consists of a metallic base body (9), which is coated with a shock-absorbing layer (10) of plastic.

3. Thigh stump endoprosthesis according to claim 2, in which the shock-absorbing layer (10) has a thickness of 3 to 10 mm.

4. Thigh stump endoprosthesis according to claim 2 or 3, in which the shock-absorbing layer (10) consists of silicone.

## Revendications

1. Endoprothèse de moignon de cuisse pour soin exoprothétique d'un patient amputé au niveau de la cuisse, dans lauelle le moignon de la cuisse (1) peut être placé sur une tige (2), à laquelle s'attachent les parties d'articulation de genou, de jarret et de pied (3) de la prothèse, présentant une tige proximale (4) pouvant être placée dans un moignon fémoral (5), la tige (4) étant au moins en partie recouverte d'une structure en filet en trois dimensions à mailles ouvertes (6), **caractérisée en ce que** la tige porte en son extrémité distale un adaptateur conique (7) permettant de coupler un substitut de condyle (8), ayant la forme (11) du condyle naturel d'une articulation de genou, à la tige (4).

2. Endoprothèse de moignon de la cuisse selon la revendication 1, dans laquelle le substitut de condyle (8) est composé d'un corps métallique (9) recouvert d'une couche amortissant les chocs (10) en matière plastique.

3. Endoprothèse de moignon de la cuisse selon la revendication 2', dans laquelle la couche amortissant les chocs (10) a une épaisseur comprise entre 3 et 10 mm.

4. Endoprothèse de moignon de la cuisse selon la revendication 2 ou 3, dans laquelle la couche amortissant les chocs (10) est en silicone.
